# EUROPEAN PATENT SPECIFICATION

(11) **EP 3 782 671 B1**
(45) Date of publication and mention of the grant of the patent: **07.07.2021**
(21) Application number: 19192317.6
(22) Date of filing: 19.08.2019
(51) Int. Cl.: A61M 1/36

(54) **METHOD FOR PRIMING AN EXTRACORPOREAL BLOOD CIRCUIT OF AN APPARATUS FOR EXTRACORPOREAL TREATMENT OF BLOOD AND APPARATUS FOR EXTRACORPOREAL TREATMENT OF BLOOD**
VERFAHREN ZUM PRIMING EINES EXTRAKORPORALEN BLUTKREISLAUFS EINER VORRICHTUNG ZUR EXTRAKORPORALEN BEHANDLUNG VON BLUT UND VORRICHTUNG ZUR EXTRAKORPORALEN BEHANDLUNG VON BLUT
PROCÉDÉ D'AMORÇAGE D'UN CIRCUIT SANGUIN EXTRACORPOREL D'UN APPAREIL DE TRAITEMENT EXTRACORPOREL DU SANG ET APPAREIL DE TRAITEMENT EXTRACORPOREL DU SANG

(43) Date of publication of application: 24.02.2021
(73) Proprietor: Gambro Lundia AB, 226 43 Lund (SE)
(72) Inventor: POUCHOULIN, Dominique, F-01390 Tramoyes (FR); BELOT, Valentin, BE-1190 FOREST (BE)
(74) Representative: PGA S.p.A., Milano, Succursale di Lugano

(56) References cited:
- WO-A1-2017/190718
- US-A1- 2006 167 400

## Description

The invention relates to a method for priming anextracorporeal blood circuit of an apparatus for extracorporeal treatment of blood and an apparatus for extracorporeal treatment of blood configured to implement such method, in particular an apparatus provided with a membrane gas exchanger for the purpose of oxygenation and/or CO₂ removal.

In the field of blood extracorporeal blood treatments and therapies, membrane gas exchangers are used for the purpose of ExtraCorporeal Membrane Oxygenation (ECMO) and/or ExtraCorporeal CO₂ Removal (ECCO₂R). While originally used in dedicated systems, development of ExtraCorporeal CO₂ Removal has recently led to the introduction of membrane gas exchangers in dialysis systems for Continuous Renal Replacement Therapy (CRRT). The CRRT systems can deliver ECCO₂R therapy (stand-alone ECCO₂R), as well as CRRT and ECCO₂R combined in the same blood circuit or other therapy combinations, e.g. liver support and ECCO₂R.

In an haemodialysis treatment a patient's blood and a treatment liquid approximately isotonic with blood flow are circulated in a respective compartment of haemodialyser, so that, impurities and undesired substances present in the blood (urea, creatinine, etc.) may migrate by diffusive transfer from the blood into the treatment liquid. The ion concentration of the treatment liquid is chosen so as to correct the ion concentration of the patient's blood. In a treatment by haemodiafiltration, a convective transfer by ultrafiltration, resulting from a positive pressure difference created between the blood side and the treatment-liquid side of the membrane of a haemodiafilter, is added to the diffusive transfer obtained by dialysis.

Before performing an extracorporeal blood treatment, the extracorporeal blood circuit of the apparatus is primed, making a priming solution, e.g. saline, flow through the blood lines. The purpose of priming the extracorporeal blood circuit is to remove air from the blood lines, the membrane gas exchanger and the dialyzer as well as to remove possible fragments of remaining sterilizing agents or other residuals from the disposables elements before connecting a patient.

Because of their membrane properties, membrane gas exchangers require specific precautions during and after priming to prevent air intake through the membrane and formation of bubbles during the following blood treatment.

For instance, it is known to position the gas exchanger device below the end of the return line during priming and to position the gas exchanger device below the patient during treatment in order to keep the circuit pressure above atmospheric pressure.

This way, the membrane gas exchanger cannot be freely positioned and the low location of said gas exchanger is not convenient for the user who has to bend for setting the gas exchanger on its holder and cannot see it when working on the user interface of the apparatus.

Document US2006167400A1 describes a blood perfusion system used in cardiopulmonary bypass procedures. The system comprises a combined oxygenator and heat exchanger. The oxygenator has an oxygenator vent tubing line from the oxygenator to a venous reservoir. The vent tubing line passes through a vent valve which is automatically opened during priming to remove air from the oxygenator. This document discloses that, by pressurizing the priming solution, coming from bags, in the oxygenator to a predetermined value, leaks in the oxygenator membrane can be detected with a liquid leak detector as fluid would transverse a leaky oxygenator membrane at a predetermined pressure.

Document EP1372759B1 describes a system for preparing and delivering gas-enriched blood. In a prime mode, the system fills a fluid supply chamber with physiologic solution and drives a piston assembly to pressurize the solution and transfer it into an atomizer chamber until appropriate level of fluid is reached. The system includes a bubble detector that interfaces with a bubble sensor to monitor the oxygen-enriched blood in a return tube for bubbles.

Document WO2017190718A1 describes an oxygenator circuit (with oxygenator, blood pump) provided with a venting device set comprising a priming liquid container, a priming compressor and a venting unit. The circuit is filled with priming liquid from the priming liquid container and the oxygenator is vented. Sensor checks whether it detects air bubbles in the priming circuit. If air bubbles are detected, the blood pump runs in pulsatile mode to deliver residual air into the oxygenator, from which the residual air can escape.

The above described prior art documents do not prevent formation of bubbles during and after priming but eliminate air from the oxygenator or from the blood lines through vent devices and/or bubble sensors.

It is therefore an object of the present invention to provide a method for priming an extracorporeal blood circuit and an apparatus for extracorporeal treatment of blood configured to reliably prevent formation of bubbles in the blood circuit due to the presence of the membrane gas exchanger.

In particular, it is an object to prevent formation of bubbles due to the presence of the membrane gas exchanger at least during priming and possibly after priming, during patient treatment.

Additionally, it is an object providing a method and an apparatus configured to prevent bubble formation which do not require any additional and peculiar component/device.

Another auxiliary object is to provide a method and an apparatus allowing a free and optionally user friendly positioning of the membrane gas exchanger.

A further auxiliary object is to provide a priming method which may be fully automated and may not require any user intervention.

### SUMMARY

At least one of the above objects is substantially reached by a method for priming an extracorporeal blood circuit of an apparatus for extracorporeal treatment of blood and by an apparatus for extracorporeal treatment of blood according to one or more of the appended claims.

The effect of the pressurization step (i.e. preventing release of air bubbles at a blood outlet of the membrane gas exchanger) may result from the forcing of some fluid into the hydrophobic pores of the membrane leading to a reduction of gas transfer, as well as from the removal of micro-air bubbles, accumulated at the membrane wall, through the membrane and before their aggregate into macro-bubbles. Later description will show such effect can be investigated in a reproducible way.

### DESCRIPTION OF THE DRAWINGS

Aspects of the invention are shown in the attached drawings, which are provided by way of non-limiting example, wherein:
Figure 1 shows a schematic diagram of an apparatus for extracorporeal treatment of blood during treatment of a patient;
Figure 2 shows the apparatus of figure 1 during a priming procedure according to one aspect of the invention;
Figure 3 shows a schematic diagram of an alternative embodiment of an apparatus for extracorporeal treatment of blood;
Figure 4 shows the apparatus of figure 1 during a priming procedure according to another aspect of the invention;
Figure 5 shows a possible embodiment of the apparatus of figures 1 and 2;
Figure 6 is a graph showing a pressure trend during priming related to an embodiment of the invention;

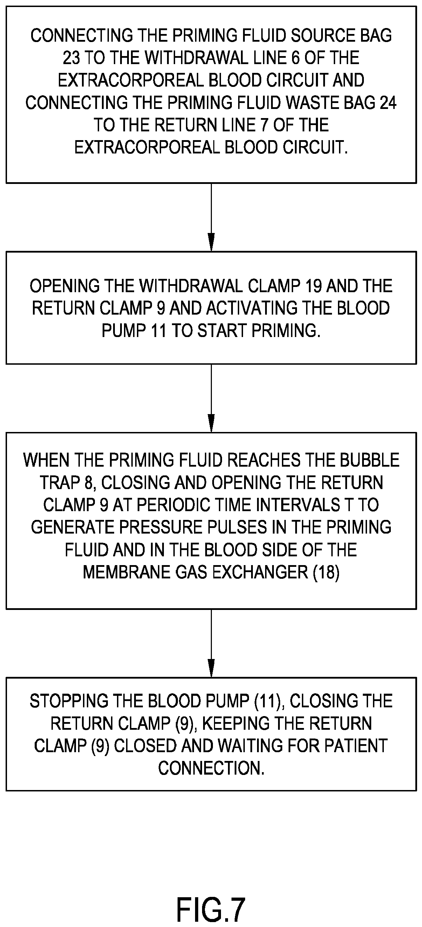

Figure 7 is a flowchart of one embodiment of a method of the invention;
Figure 8 is a graph showing correlation between intensity of intensity of pressure peaks and bubbling free time during priming;
Figure 9 is a graph showing correlation between pressurization time and bubbling free time during priming.

### DETAILED DESCRIPTION

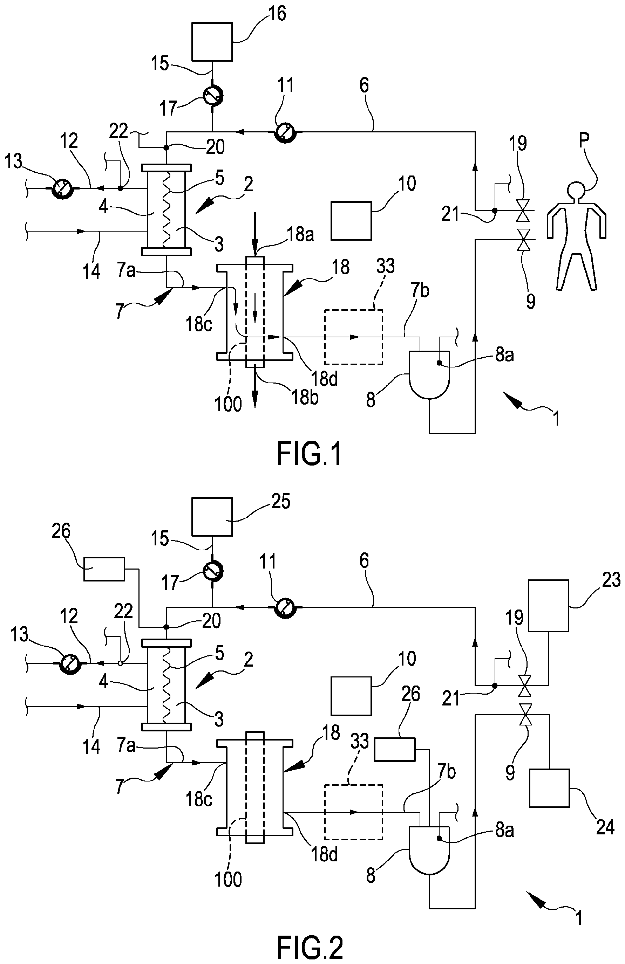

Non-limiting embodiments of an apparatus 1 for extracorporeal treatment of blood - which may implement innovative aspects of the invention - are shown in figures 1 to 5. In below description and in figures 1 to 5 same components are identified by same reference numerals.

In figure 1 it is represented an apparatus for the extracorporeal treatment of blood 1 comprising a blood treatment unit 2 (such as an hemofilter, an ultrafilter, an hemodiafilter, a dialyzer, a plasmafilter and the like) having a primary chamber 3 and a secondary chamber 4 separated by a semi-permeable membrane 5; depending upon the treatment, the membrane 5 of the blood treatment unit 2 may be selected to have different properties and performances. A blood withdrawal line 6 is connected to an inlet of the primary chamber 3, and a blood return line 7 is connected to an outlet of the primary chamber 3. In use, the blood withdrawal line 6 and the blood return line 7 are connected to a needle or to a catheter or other access device (not shown) which is then placed in fluid communication with the patient P vascular system, such that blood may be withdrawn through the blood withdrawal line 6, flown through the primary chamber 3 and then returned to the patient's vascular system through the blood return line 7. An air separator, such as a deaeration chamber 8, may be present on the blood return line 7. Moreover, a safety return clamp 9 controlled by a control unit 10 may be present on the blood return line 7, downstream the deaeration chamber 8. A bubble sensor 8a, for instance associated to the deaeration chamber 8 or coupled to a portion of the line 7 between the deaeration chamber 8 and the return clamp 9 may be present: if present, the bubble sensor 8a is connected to the control unit 10 and sends to the control unit 10 signals for the control unit 10 to cause closure of the return clamp 9 in case one or more bubbles above certain safety thresholds are detected. The blood flow through the blood lines is controlled by a blood pump 11, for instance a peristaltic blood pump, acting either on the blood withdrawal line 6 or on the blood return line 7. The embodiment of figures 1 and 2 shows the blood pump 11 coupled to a pump section of the withdrawal line 6. An operator may enter a set value for the blood flow rate Q_{B} through a user interface and the control unit 10, during treatment, is configured to control the blood pump 11 based on the set blood flow rate Q_{B}.

The control unit 10 may comprise a digital processor (CPU) with memory (or memories), an analogical type circuit, or a combination of one or more digital processing units with one or more analogical processing circuits. In the present description and in the claims it is indicated that the control unit 10 is "configured" or "programmed" to execute certain steps: this may be achieved in practice by any means which allow configuring or programming the control unit 10. For instance, in case of a control unit 10 comprising one or more CPUs, one or more programs are stored in an appropriate memory: the program or programs containing instructions which, when executed by the control unit 10, cause the control unit 10 to execute the steps described and/or claimed in connection with the control unit 10. Alternatively, if the control unit 10 is of an analogical type, then the circuitry of the control unit 10 is designed to include circuitry configured, in use, to process electric signals such as to execute the control unit 10 steps herein disclosed.

An effluent fluid line or spent dialysate line 12 is connected, at one end, to an outlet of the secondary chamber 4 and, at its other end, to a waste which may be a discharge conduit or an effluent fluid container collecting the fluid extracted from the secondary chamber. An effluent pump 13 that operates on the effluent fluid line 12 under the control of the control unit 10 to regulate the flow rate Q_{eff} across the effluent fluid line.

The apparatus of figure 1 includes a dialysis line 14 connected at one end with a liquid inlet and at its other end with the inlet of the secondary chamber 4 of the treatment unit 2 for supplying fresh dialysis liquid to the secondary chamber 4. A dialysis fluid pump, not shown, is operative on the dialysis fluid line 14 under the control of the control unit 10, to supply fluid from a dialysis liquid container to the secondary chamber 4 at a flow rate Q_{dial}.

The embodiment of figure 1 presents an infusion line 15 connected to the blood withdrawal line 6 between the blood pump 11 and the treatment unit 2. This infusion line 15 supplies replacement fluid from an infusion fluid container 16 connected at one end of the infusion line 15. Note that, alternatively or in addition to the infusion line 15, the apparatus of figure 1 may include a post-dilution fluid line (not shown) connecting an infusion fluid container to the blood return line 7. Furthermore, an infusion pump 17 operates on the infusion line 15 to regulate the flow rate Qᵣₑₚ through the infusion line 15. Note that in case of two infusion lines (pre-dilution and post-dilution) each infusion line may be provided with a respective infusion pump.

The apparatus for the extracorporeal treatment of blood 1 further comprises a membrane gas exchanger 18 placed on the blood return line 7, i.e. downstream of the treatment unit 2 with respect to a flow direction of blood during treatment. The membrane gas exchanger 18 comprises a gas permeable membrane 100 separating a blood side and a gas side. A first section 7a of the blood return line 7 coming from the treatment unit 2 is connected to a blood inlet 18c of the blood side of the membrane gas exchanger 18 and a second section 7b of the blood return line 7, connected to the needle or to the catheter, is connected to a blood outlet 18d of the blood side of the membrane gas exchanger 18. The gas side of the membrane gas exchanger 18 is provided with a respective gas inlet 18a and gas outlet 18b for ventilating gas (e.g. air or oxygen).

The internal structure of the membrane gas exchanger 18 may be per se known. The gas permeable membrane 100 may comprise a plurality of hollow fibers. The ventilating gas (e.g. oxygen, air) is passed through the inside (gas side) of the hollow fibers, while the blood is passed around (blood side) the hollow fibers to accomplish gas exchange by diffusion. The membrane gas exchanger 18 is operatively coupled to the extracorporeal blood circuit to exchange gas with blood flowing in the extracorporeal blood circuit. The membrane gas exchanger 18 may be an oxygenator and/or a CO₂ remover. For example, oxygen diffuses from the gas side into the blood and carbon dioxide CO₂ diffuses from the blood side into the gas for disposal. The apparatus 1 of figure 1 can deliver stand-alone CO₂ removal as well as dialysis and CO₂ removal combined in the same blood circuit.

The apparatus 1 shown in figures 1 and 2 is also provided with a safety withdrawal clamp 19 controlled by the control unit 10 and present on the blood withdrawal line 6 and upstream of the blood pump 11.

The blood withdrawal line 6, the blood return line 7, the first chamber 3 of the treatment unit 2 and the blood side of the membrane gas exchanger 18 form part of an extracorporeal blood circuit of the apparatus 1. The effluent fluid line 12, the dialysis fluid line 14, the fluid chamber 4 of the treatment unit 2 form part of a fluid circuit of the apparatus 1. The infusion line 15 is connected to the blood circuit between the return clamp 9 and the blood pump 11. In figures 1 and 2 the infusion line 15 is connected to the blood circuit between the blood pump 11 and the blood treatment unit 2.

Pressure pods may also be present on the blood circuit and on the fluid circuit to monitor liquid/blood pressures. Each pressure pod comprises a hollow body with an intermediate flexible membrane which delimits a gas chamber and a liquid/blood chamber with inlet and outlet for connection to the blood circuit or to the fluid circuit.

The apparatus 1 shown in figures 1 and 2 comprises a treatment unit pressure pod 20 placed on the blood withdrawal line 6 just upstream of the treatment unit 2, an access pressure pod 21 placed on the blood withdrawal line 6 just downstream of the access device and of the patient P, an effluent pressure pod 22 placed on the effluent line 12 between the treatment unit 2 and the effluent pump 13.

The blood pump 11, the effluent pump 13, the infusion pump 17 and possible other pumps (not shown) are operatively connected to the control unit 10 which controls said pumps. The control unit 10 is also operatively connected to sensors (like flow sensors) on the blood circuit and/or fluid circuit and, in particular, to the pressure pods 20, 21, 22 and the bubble sensor 8a. The control unit 10 is also operatively connected to clamps and valves, like the return clamp 9 and the withdrawal clamp 19. The control unit 10 is also connected to the user interface, not shown, for instance a graphic user interface, which receives operator's inputs and displays the apparatus outputs. For instance, the graphic user interface may include a touch screen, a display screen and hard keys for entering user's inputs or a combination thereof. During extracorporeal blood treatment, the control unit 10 is configured to control at least the pumps 11, 13, 17 to make sure that a prefixed patient fluid removal is achieved in the course of a treatment time, as required by a prescription provided to the control unit 10, e.g. via the user interface.

A blood warming device 33 may optionally be place on the blood return line 7 between the membrane gas exchanger 18 and the deaeration chamber 8.

The apparatus 1 of figures 1 and 2 is configured to deliver Continuous Renal Replacement Therapy (CRRT) in combination with ECCO₂R therapy or ECCO₂R therapy alone.

The control unit 10 is also configured for commanding execution of a task for priming the extracorporeal blood circuit before treatment of a patient, according also to the method of the present invention.

A configuration of the apparatus of figure 1 for priming the blood circuit is shown in figure 2. A priming fluid source bag 23 (e.g. saline bag) is connected to the withdrawal line 6 of the blood circuit. A priming fluid waste bag 24 is connected to the return line 7 of the extracorporeal blood circuit. A further priming fluid source bag 25 may be connected to the infusion line 15. An air pump 26 may be connected to the gas chamber of the treatment unit pressure pod 20. An air pump 26 may also be connected to the upper part of the deaeration chamber 8 allowing level adjustement in said deaeration chamber.

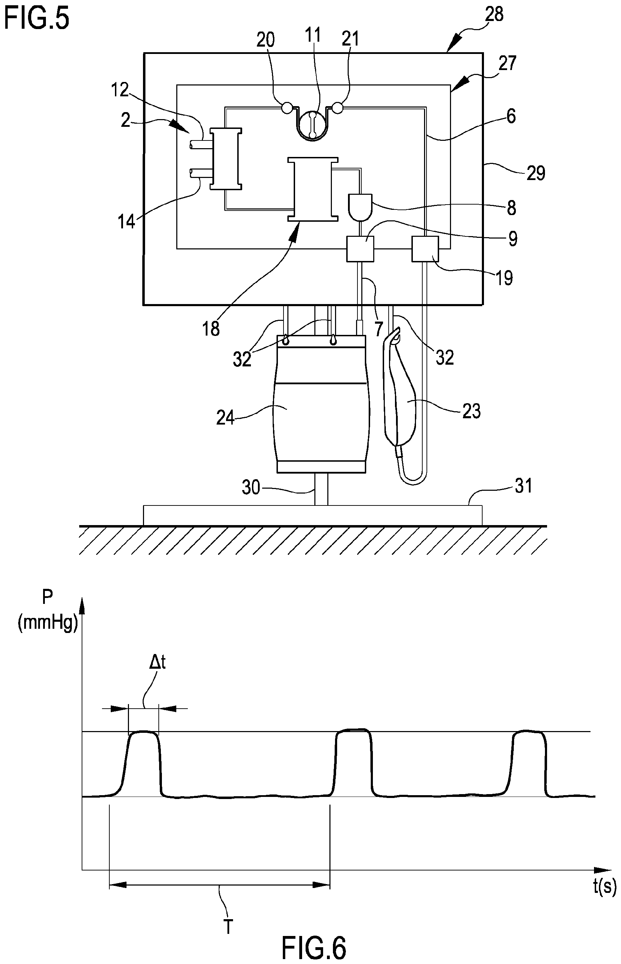

Figure 5 shows a possible embodiment of the apparatus of figures 1 and 2, wherein the extracorporeal blood treatment unit 2, the membrane gas exchanger 18 and at least part of the extracorporeal blood circuit are part of a disposable cartridge 27 mounted on a supporting frame 28.

The supporting frame 28 comprises a casing 29 supported by an upright 30 with a support base 31 configured to rest on the ground. The casing 29 supports and/or houses mechanical and/or electronic devices of the apparatus 1, such as the control unit 10, the blood pump 11, the return clamp 9, the withdrawal clamp 19, the pressure sensors to be connected to pressure pods 20, 21, etc.. The casing 29 comprises carrier for the cartridge 27, not visible, and it is further provided with supporting elements 32, such as hooks, for hanging fluid bags.

Figure 5 shows the apparatus 1 in the priming configuration in which the priming fluid source bag 23 and the priming fluid waste bag 24 hang under the casing 29. The membrane gas exchanger 18 is located next to the blood treatment unit 2 and substantially at the same height of the blood treatment unit 2. The priming fluid source bag 23 and the priming fluid waste bag 24 are placed below the membrane gas exchanger 18.

In order to prime the extracorporeal blood circuit, the return clamp 9 and the withdrawal clamp 19 are opened and the blood pump 11 is activated to make the priming fluid flow from the priming fluid source bag 23 towards the priming fluid waste bag 24 and flowing through the primary chamber 3 of the blood treatment unit 2 and the blood side of the membrane gas exchanger 18. During priming, no gas flows through the gas side of the membrane gas exchanger 18.

Once the priming fluid fills the blood side of the membrane gas exchanger 18, optionally when the priming fluid reaches the deaeration chamber 8, the return clamp 9 is closed and reopened while the blood pump 11 keeps working, in order to generate a transitory pressurization step in the priming fluid and in the blood side of the membrane gas exchanger 18.

In an embodiment of the method or task for priming, the return clamp 9 is repeatedly closed and opened in order to generate a plurality of transitory pressurization steps in the priming fluid and in the blood side of the membrane gas exchanger 18. The generation of one or more pressurization step/s may be repeated several times during priming. This prevents release of air bubbles at the outlet of the membrane gas exchanger 18.
The effect of the pressurization step may result from the forcing of some fluid into the hydrophobic pores of the membrane leading to a reduction of gas transfer, as well as from the removal of micro-air bubbles, accumulated at the membrane wall, through the membrane and before their aggregate into macro-bubbles.

For instance, when the priming fluid reaches the deaeration chamber 8, a first series of pressurization steps may be actuated by intermittently closing the return clamp 9 at periodic time intervals T.

By closing and opening the return clamp 9, to generate pressurization step or steps, a portion of the blood circuit placed downstream of the membrane gas exchanger 18 with respect to a flow direction of the priming fluid is occluded. In a variant of the method, the return clamp 9 may be partially closed in order to restrict the portion of the blood circuit placed downstream of the membrane gas exchanger 18.

According to a different embodiment for generating the pressurization step or steps, after that the priming fluid from the priming fluid source bag 23 has reached the deaeration chamber 8, the blood pump 11 is stopped, the return clamp 9 is closed and the infusion pump 17 is intermittently activated to pump priming fluid from the further priming fluid source bag 25 through the infusion line 15 and into the extracorporeal blood circuit and to generate said pressurization step/s in the membrane gas exchanger 18.

According to a further different embodiment for generating the pressurization step or steps, after that the priming fluid from the priming fluid source bag 23 has reached the deaeration chamber 8, while the blood pump keeps working, the air pump 26 connected to the treatment unit pressure pod 20 is activated intermittently to generate pressure pulses in the air chamber of the treatment unit pressure pod 20 while blood pump 11 is stopped and the return clamp 9 is closed. The pressure pulses in the air chamber pushes and deforms the intermediate flexible membrane which transfers said pressure pulses to the priming fluid in the blood chamber of the treatment unit pressure pod 20 and in the blood treatment circuit.

According to a different embodiment for generating the pressurization step or steps, the blood pump 11 is stopped, the return clamp 9 is closed and the air pump 26 connected to the deaeration chamber 8 is activated intermittently to generate pressure pulses in the upper part of the deaeration chamber 8 and into the priming fluid in the lower part of said deaeration chamber 8.

Optionally, at the end of priming and before patient connection, the blood pump 11 is still motionless while the return clamp 9 placed on a blood return line 7 and downstream of the membrane gas exchanger 18 is kept closed, while, optionally, the blood pump 11, the infusion pump 17, the dialysate pump 13 or air pump 26 are activated to build up some positive pressure level.

Even if, like in figure 5, the membrane gas exchanger 18 is located close to the blood treatment unit 2 and substantially at the same height of the blood treatment unit 2, the priming fluid source bag 23 and the priming fluid waste bag 24 and, optionally, also the further priming fluid source bag 25 (not shown in figure 5) are placed below the membrane gas exchanger 18, bubble formation is prevented. Therefore, free and user friendly positioning of the membrane gas exchanger 18 and of the bags is allowed and no specific component designed to control bubbling during priming is required.

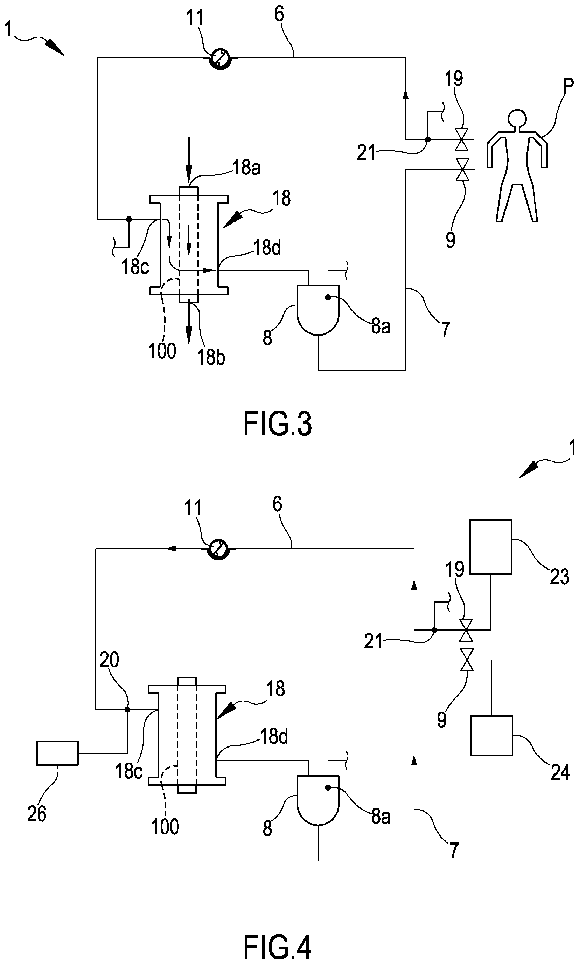

Figures 3 and 4 show a different embodiment of the extracorporeal blood treatment 1 during patient treatment (figure 3) and priming sequence (figure 4). The same reference numerals for the same elements of figures 1 and 2 have been used. The extracorporeal blood treatment 1 of figure 3 and 4 does not comprise the blood treatment unit 2 but it's only equipped with the membrane gas exchanger 18. The membrane gas exchanger 18 is the only device in the circuit and the apparatus 1 is configured to deliver only ECCO₂R therapy (stand-alone ECCO₂R).

Priming of the extracorporeal blood circuit and generation of transitory pressurization step/s may be accomplished as in the apparatus of figures 1 and 3 through the return clamp 9 and/or the air pump 26 connected to the pressure pod 20 placed upstream of the membrane gas exchanger 18.

The control unit 10 is configured to control the pressurizations step/s and, optionally, a time length Δt of each pressurization step or of each pressurization step. The pressurization step/s may be fully automated and may not require any user intervention.

According to some embodiments, the time length Δt is fixed. According to other embodiments, the time length Δt is function of one or more parameters. By way of example, the time length Δt may be function of a measured return pressure captured through the bubble sensor 8a and/or a treatment unit pressure captured through the treatment unit pressure pod 20 and/or an effluent pressure captured through the effluent pressure pod 22.

The time length Δt of each pressurization step may be between 2s and 30s, optionally between 5s and 10s, and each time interval T between one pressurization step and the following may be between 10s and 100s, optionally between 20s and 80s, optionally between 40s and 60s. A maximum pressure Pₘₐₓ at the membrane gas exchanger 18 during the pressurization step or steps may be between 100mmHg and 1000mmHg, optionally between 400mmHg and 600mmHg. At the end of priming sequence and before patient connection, a pressure in the blood circuit and in the blood side of the membrane gas exchanger 18 is kept between 20mmHg and 400mmHg, optionally between 50mmHg and 100mmHg.

Analysis of the impact of the maximum pressure Pₘₐₓ and of the time length Δt of the pressurization step on the bubble formation at the membrane gas exchanger outlet 18d was performed.

Used materials, samples and parameters were the following:
- PrisMax extracorporeal blood treatment monitor;
- PrismaFlex set cartridge equipped with membrane gas exchanger arm;
- three samples of membrane gas exchanger S1, S2 and S3;
- pressure sensor with data logging;
- saline solution as priming fluid;
- room temperature;
- fixed flow rate and fixed position of priming waste/collection bag.

### Definitions

- T_{bb}:: time in seconds for air bubbles to be seen back at the membrane gas exchanger outlet 18d after a pressurization step;
- Pₘₐₓ:: maximum pressure or pressure peak in mmHg recorded through a pressurization step;
- Tₚ:: time in seconds with pressure above +300 mmHg during a pressurization step;
- IntP:: integral of the pressure-time signal during the pressurization step expressed in mmHg x s;
- P_{range}:: pressure range in mmHg of Pₘₐₓ.

The time length Δt mentioned above is correlated to Tₚ and IntP.

The investigation was split in two parts.

### Part 1

Impact of the maximum pressure Pₘₐₓ on the T_{bb} has been investigated.

Next Tables 1, 2 and 3 report for T_{bb}, Pₘₐₓ and P_{range} recorded throughout all pressurization steps/challenges.

**Table 1 - S1**

| Challenge | 1 | 6 | 11 | 12 | 3 | 5 | 8 | 9 | 2 | 4 | 7 | 10 |
|---|---|---|---|---|---|---|---|---|---|---|---|---|
| Pₘₐₓ | 162 | 175 | 193 | 139 | 266 | 225 | 217 | 338 | 464 | 674 | 682 | 412 |
| IntP | 692 | 579 | 1117 | 242 | 1331 | 670 | 655 | 1216 | 1410 | 1712 | 2147 | 1623 |
| P_{range} | Pₘₐₓ < 200 | | | | 200 < Pₘₐₓ < 400 | | | | 400 < Pₘₐₓ < 700 | | | |
| T_{bb} | 60 | 50 | 40 | 30 | 70 | 70 | 60 | 50 | 110 | 80 | 100 | 70 |
| Mean & Std | 45±13 | | | | 63±10 | | | | 90±18 | | | |

**Table 2 - S2**

| Challenge | 2 | 6 | 9 | 12 | 4 | 5 | 8 | 10 | 1 | 2 | 7 | 11 |
|---|---|---|---|---|---|---|---|---|---|---|---|---|
| Pₘₐₓ | 102 | 145 | 194 | 129 | 229 | 319 | 317 | 332 | 446 | 619 | 492 | 617 |
| IntP | 430 | 510 | 1002 | 307 | 1142 | 1104 | 1008 | 1162 | 1409 | 1495 | 2200 | 2065 |
| P_{range} | Pₘₐₓ < 200 | | | | 200 < Pₘₐₓ < 400 | | | | 400 < Pₘₐₓ < 700 | | | |
| T_{bb} | 15 | 15 | 35 | 10 | 30 | 30 | 40 | 35 | 100 | 50 | 70 | 60 |
| Mean & Std | 19±11 | | | | 34±5 | | | | 70±22 | | | |

**Table 3 - S3**

| Challenge | 2 | 5 | 10 | 12 | 1 | 4 | 7 | 11 | 3 | 6 | 8 | 9 |
|---|---|---|---|---|---|---|---|---|---|---|---|---|
| Pₘₐₓ | 152 | 137 | 152 | 199 | 331 | 300 | 316 | 247 | 650 | 538 | 630 | 403 |
| IntP | 431 | 791 | 830 | 411 | 870 | 861 | 1038 | 653 | 2280 | 1324 | 1409 | 955 |
| P_{range} | Pₘₐₓ < 200 | | | | 200 < Pₘₐₓ < 400 | | | | 400 < Pₘₐₓ < 700 | | | |
| T_{bb} | 30 | 26 | 25 | 20 | 55 | 40 | 40 | 30 | 85 | 55 | 70 | 50 |
| Mean & Std | 25±4 | | | | 41±10 | | | | 65±16 | | | |

### Comments

Four challenges were performed with peak pressure < 200 mmHg, four challenges with 200 < peak pressure < 400 mmHg and four challenges with 400 < peak pressure < 700 mmHg for each of the three tested Falcon gas exchangers.

Mean return pressure level was about -15 mmHg during priming (outside challenges).

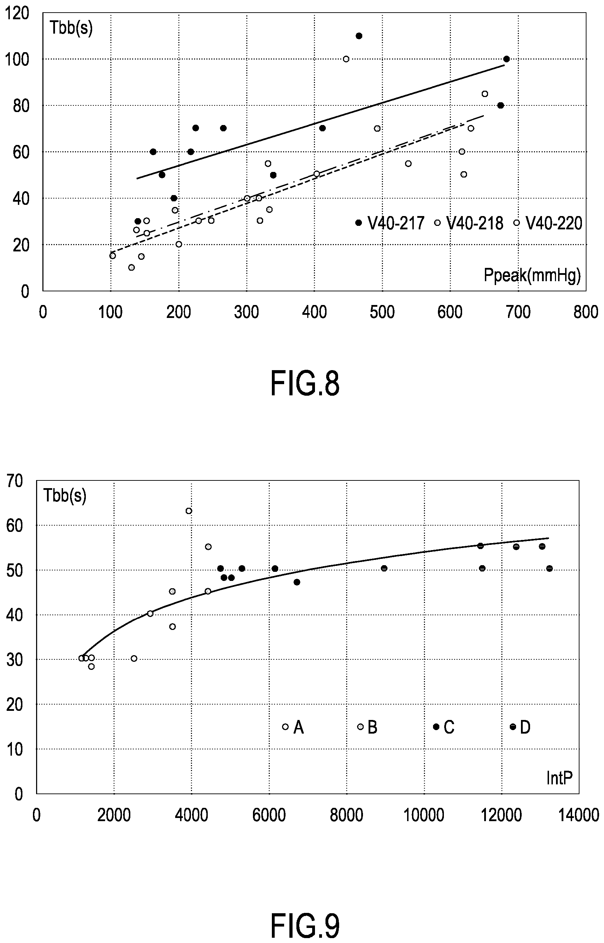

Tables 1, 2 and 3 and figure 8 show that bubbling free time T_{bb} increases when pressurization pressure is increased.

### Part 2

Impact of IntP and Tₚ on the T_{bb} has been investigated.

Next Tables 4 to 6 report for T_{bb}, Pₚₑₐₖ & P_{range} parameters recorded throughout all pressurization challenges.
Challenges are identified as follows: X_y with X and y relating to tested condition ID (A, B, C or D) and test chronology order, respectively.
A to D test conditions are referenced in reference to the pressurization time (see Tₚ parameter).

**Table 4 - S1**

| Challenge | A_5 | A_7 | B_6 | B_8 | C_2 | C_3 | D_1 | D_4 |
|---|---|---|---|---|---|---|---|---|
| Pₚₑₐₖ | 614 | 616 | 609 | 560 | 553 | 491 | 590 | 616 |
| IntP | 1418 | 1399 | 4410 | 3490 | 6139 | 4741 | 11440 | 12375 |
| Tₚ | 2.2 | 2.2 | 7.4 | 6.2 | 12.4 | 9.0 | 20.8 | 20.0 |
| T_{bb} | 30 | 28 | 45 | 45 | 50 | 50 | 55 | 55 |
| Mean | 29 | | 45 | | 50 | | 55 | |

**Table 5 - S2**

| Challenge | A_3 | A_6 | B_1 | B_4 | C_5 | C_7 | D_2 | D_8 |
|---|---|---|---|---|---|---|---|---|
| Pₚₑₐₖ | 537 | 606 | 562 | 597 | 581 | 532 | 601 | 514 |
| IntP | 1409 | 2526 | 3939 | 3439 | 6720 | 5027 | 11483 | 8951 |
| Tₚ | 2.4 | 4.2 | 5.2 | 6.0 | 11.4 | 9.8 | 18.8 | 17.2 |
| T_{bb} | 30 | 30 | 63 | 37 | 47 | 48 | 50 | 50 |
| Mean | 30 | | 50 | | 48 | | 50 | |

**Table 6 - S3**

| Challenge | A_2 | A_7 | B_1 | B_6 | C_3 | C_5 | D_4 | D_8 |
|---|---|---|---|---|---|---|---|---|
| Pₚₑₐₖ | 454 | 602 | 598 | 646 | 592 | 510 | 607 | 609 |
| IntP | 1184 | 1255 | 4439 | 2923 | 5280 | 4834 | 13208 | 13028 |
| Tₚ | 2.0 | 2.2 | 7.4 | 4.8 | 8.6 | 9.2 | 21.4 | 21.8 |
| T_{bb} | 30 | 30 | 55 | 40 | 50 | 48 | 50 | 55 |
| Mean | 30 | | 48 | | 49 | | 53 | |

### Comments

Mean pressure level was about -27 mmHg in run mode conditions without challenges; that can explain slightly lower Tbb values from Part II versus Part I testing.
Tables 4, 5 and 6 and Figure 9 illustrate little dependence of bubbling free time T_{bb} on pressurization time, with sort of threshold effect when time reaches about 5 seconds (or IntP about 4000 mmHg x s).

This investigation documents that the pressure level reached during pressurization step is the main physical parameter controlling the time during which bubbling is inhibited afterwards.

### Example of priming sequence

- connecting the priming fluid source bag 23 (e.g. saline bag) to the withdrawal line 6 of the extracorporeal blood circuit;
- connecting the priming fluid waste bag 24 to the return line 7 of the extracorporeal blood circuit;
- opening the withdrawal clamp 19 and the return clamp 9 and activating the blood pump 11 to start priming;
- when the priming fluid reaches the deaeration chamber 8, closing and opening the return clamp 9 at periodic time intervals T to generate pressure pulses in the priming fluid and in the blood side of the membrane gas exchanger (18);
- stopping the blood pump (11), closing the return clamp (9), keeping the return clamp (9) closed and waiting for patient connection.

While the invention has been described in connection with what is presently considered to be the most practical and preferred embodiments, it is to be understood that the invention is not to be limited to the disclosed embodiments, but on the contrary, is intended to cover various modifications included within the scope of the appended claims.

## Claims

1. A method for priming an extracorporeal blood circuit of an apparatus for extracorporeal treatment of blood, wherein the apparatus for extracorporeal treatment of blood comprises:
optionally a blood treatment unit (2);
an extracorporeal blood circuit, optionally coupled to the blood treatment unit (2);
a blood pump (11) configured to be coupled to a pump section of the extracorporeal blood circuit;
a membrane gas exchanger (18) operatively coupled to the extracorporeal blood circuit to exchange gas with blood flowing in the extracorporeal blood circuit, wherein the membrane gas exchanger (18) comprises a blood side in fluid communication with the blood circuit and a gas side;
wherein the method comprises:
- feeding a priming fluid in the extracorporeal blood circuit and into the blood side of the membrane gas exchanger (18);
- generating a transitory pressurization step in the priming fluid flowing in the blood circuit and in the blood side of the membrane gas exchanger (18) to prevent release of air bubbles at a blood outlet (18d) of the membrane gas exchanger (18).

2. The method according to claim 1, comprising: repeating the transitory pressurization step during priming, optionally at periodic intervals.

3. The method according to claim 1 or 2, wherein a maximum pressure (Pₘₐₓ) at the membrane gas exchanger (18) during the pressurization step or steps is between 100mmHg and 1000mmHg.

4. The method according to claim 1, 2 or 3, wherein a time length (Δt) of the pressurization step or of each pressurization step is between 2s seconds and 30s seconds, optionally between 5s and 10s.

5. The method according to any of claims 1 to 4, wherein generating the transitory pressurization step comprises:
restricting or occluding transiently a portion of the extracorporeal blood circuit placed downstream of the membrane gas exchanger (18) with respect to a flow direction of the priming fluid.

6. The method according to any of claims 1 to 5, wherein generating the transitory pressurization step comprises: keeping the blood pump (11) working and closing a clamp (9) placed downstream of the membrane gas exchanger (18) with respect to a flow direction of the priming fluid.

7. The method according to any of claims 1 to 5, wherein generating the transitory pressurization step is actuated through an infusion line (15) and an infusion pump (17) coupled or configured to be coupled to a pump section of the infusion line (15) .

8. The method according to any of claims 1 to 5, wherein generating the transitory pressurization step is actuated through a deaeration chamber (8) placed on the extracorporeal blood circuit and an air pump (26) connected to the deaeration chamber (8) .

9. The method according to any of claims 1 to 8, wherein, at the end of priming and before patient connection, a pressure in the blood circuit and in the blood side of the membrane gas exchanger (18) is kept between 20mmHg and 400mmHg, optionally between 50mmHg and 100mmHg.

10. An apparatus for extracorporeal treatment of blood comprising:
optionally a blood treatment unit (2);
an extracorporeal blood circuit, optionally coupled to the blood treatment unit (2);
a blood pump (11) configured to be coupled to a pump section of the extracorporeal blood circuit;
a membrane gas exchanger (18) operatively coupled to the extracorporeal blood circuit to exchange gas with blood flowing in the extracorporeal blood circuit;
a control unit (10) configured for commanding execution of a task for priming the extracorporeal blood circuit, said task comprising the following steps:
- feeding a priming fluid in the extracorporeal blood circuit and into the blood side of the membrane gas exchanger (18);
- generating a transitory pressurization step in the priming fluid flowing in the blood circuit and in the blood side of the membrane gas exchanger (18) to prevent release of air bubbles at a blood outlet (18d) of the membrane gas exchanger (18) .

11. The apparatus of claim 10, wherein said task comprises:
repeating the transitory pressurization step during priming, optionally at periodic intervals.

12. The apparatus of claim 10 or 11, wherein, in order to generate the transitory pressurization step, said task comprises: keeping the blood pump (11) working and closing, optionally repeatedly closing, a clamp placed downstream of the membrane gas exchanger (18) with respect to a flow direction of the priming fluid, optionally a return clamp (9).

13. The apparatus of claims 10, 11 or 12, wherein the membrane gas exchanger (18) is located next to the blood treatment unit (2).

14. The apparatus of any of claims 10 to 13, wherein the membrane gas exchanger (18) is located substantially at the same height of the blood treatment unit (2), wherein, optionally, the priming fluid waste bag () is placed substantially at the same height of the membrane gas exchanger (18) or below the membrane gas exchanger (18).

15. The apparatus of any of claims 10 to 14, comprising a disposable cartridge (27) and wherein the disposable cartridge () comprises the membrane gas exchanger (18), optionally the blood treatment unit (2), and at least part of the extracorporeal blood circuit.

## Patentansprüche

1. Verfahren zum Priming eines extrakorporalen Blutkreislaufs einer Vorrichtung zur extrakorporalen Behandlung von Blut, wobei die Vorrichtung zur extrakorporalen Behandlung von Blut umfasst:
optional eine Blutbehandlungseinheit (2);
einen extrakorporalen Blutkreislauf, der optional mit der Blutbehandlungseinheit (2) gekoppelt ist;
eine Blutpumpe (11), die dazu ausgelegt ist, mit einem Pumpenabschnitt des extrakorporalen Blutkreislaufs gekoppelt zu werden;
einen Membrangasaustauscher (18), der funktional mit dem extrakorporalen Blutkreislauf gekoppelt ist, um Gas durch Blut, das in dem extrakorporalen Blutkreislauf fließt, auszutauschen, wobei der Membrangasaustauscher (18) eine Blutseite in Fluidverbindung mit dem Blutkreislauf und eine Gasseite umfasst;
wobei das Verfahren umfasst:
- Einbringen eines Priming-Fluids in den extrakorporalen Blutkreislauf und in die Blutseite des Membrangasaustauschers (18);
- Erzeugen eines Schrittes der vorübergehenden Druckbeaufschlagung in dem Priming-Fluid, das in dem Blutkreislauf fließt, und in der Blutseite des Membrangasaustauschers (18), um die Freisetzung von Luftblasen an einem Blutauslass (18d) des Membrangasaustauschers (18) zu verhindern.

2. Verfahren nach Anspruch 1, umfassend: Wiederholen des Schrittes der vorübergehenden Druckbeaufschlagung während des Primings, optional in periodischen Intervallen.

3. Verfahren nach Anspruch 1 oder 2, wobei ein maximaler Druck (Pₘₐₓ) an dem Membrangasaustauscher (18) während des Druckbeaufschlagungsschrittes oder der - schritte zwischen 100 mmHg und 1000 mmHg liegt.

4. Verfahren nach Anspruch 1, 2 oder 3, wobei eine Zeitspanne (Δt) des Druckbeaufschlagungsschrittes oder jedes Druckbeaufschlagungsschrittes zwischen 2s und 30s, optional zwischen 5s und 10s liegt.

5. Verfahren nach einem der Ansprüche 1 bis 4, wobei das Erzeugen des Schrittes der vorübergehenden Druckbeaufschlagung umfasst: Begrenzen oder vorübergehend Verschließen eines Abschnitts des extrakorporalen Blutkreislaufs, der dem Membrangasaustauscher (18) mit Bezug auf eine Strömungsrichtung des Priming-Fluids nachgelagert platziert ist.

6. Verfahren nach einem der Ansprüche 1 bis 5, wobei das Erzeugen des Schrittes der vorübergehenden Druckbeaufschlagung umfasst: Amlaufenhalten der Blutpumpe (11) und Schließen einer Klemme (9), die dem Membrangasaustauscher (18) mit Bezug auf eine Strömungsrichtung des Priming-Fluids nachgelagert positioniert ist.

7. Verfahren nach einem der Ansprüche 1 bis 5, wobei das Erzeugen des Schrittes der vorübergehenden Druckbeaufschlagung durch eine Infusionslinie (15) und eine Infusionspumpe (17) ausgelöst wird, die mit einem Pumpenabschnitt der Infusionslinie (15) gekoppelt ist oder dazu ausgelegt ist, damit gekoppelt zu werden.

8. Verfahren nach einem der Ansprüche 1 bis 5, wobei das Erzeugen des Schrittes der vorübergehenden Druckbeaufschlagung durch eine Entlüftungskammer (8), die an dem extrakorporalen Blutkreislauf platziert ist, und eine Luftpumpe (26), die mit der Entlüftungskammer (8) verbunden ist, ausgelöst wird.

9. Verfahren nach einem der Ansprüche 1 bis 8, wobei am Ende des Primings und vor der Verbindung mit dem Patienten ein Druck in dem Blutkreislauf und in der Blutseite des Membrangasaustauschers (18) zwischen 20 mmHg und 400 mmHg, optional zwischen 50 mmHg und 100 mmHg gehalten wird.

10. Vorrichtung zur extrakorporalen Behandlung von Blut, umfassend:
optional eine Blutbehandlungseinheit (2);
einen extrakorporalen Blutkreislauf, der optional mit der Blutbehandlungseinheit (2) gekoppelt ist;
eine Blutpumpe (11), die dazu ausgelegt ist, mit einem Pumpenabschnitt des extrakorporalen Blutkreislaufs gekoppelt zu werden;
einen Membrangasaustauscher (18), der funktional mit dem extrakorporalen Blutkreislauf gekoppelt ist, um Gas durch Blut, das in dem extrakorporalen Blutkreislauf fließt, auszutauschen;
eine Steuereinheit (10), die zum Anweisen der Ausführung einer Aufgabe zum Priming des extrakorporalen Blutkreislaufs ausgelegt ist, wobei die Aufgabe die folgenden Schritte umfasst:
- Einbringen eines Priming-Fluids in den extrakorporalen Blutkreislauf und in die Blutseite des Membrangasaustauschers (18);
- Erzeugen eines Schrittes der vorübergehenden Druckbeaufschlagung in dem Priming-Fluid, das in dem Blutkreislauf fließt, und in der Blutseite des Membrangasaustauschers (18), um die Freisetzung von Luftblasen an einem Blutauslass (18d) des Membrangasaustauschers (18) zu verhindern.

11. Vorrichtung nach Anspruch 10, wobei die Aufgabe umfasst: Wiederholen des Schrittes der vorübergehenden Druckbeaufschlagung während des Primings, optional in periodischen Intervallen.

12. Vorrichtung nach Anspruch 10 oder 11, wobei zum Erzeugen des Schrittes der vorübergehenden Druckbeaufschlagung die Aufgabe umfasst: Amlaufenhalten der Blutpumpe (11) und Schließen, optional wiederholtes Schließen, einer Klemme die dem Membrangasaustauscher (18) mit Bezug auf eine Strömungsrichtung des Priming-Fluids, optional einer Rückführklemme (9), nachgelagert positioniert ist.

13. Vorrichtung nach Anspruch 10, 11 oder 12, wobei der Membrangasaustauscher (18) neben der Blutbehandlungseinheit (2) angeordnet ist.

14. Vorrichtung nach einem der Ansprüche 10 bis 13, wobei der Membrangasaustauscher (18) im Wesentlichen auf derselben Höhe der Blutbehandlungseinheit (2) angeordnet ist, wobei optional die Priming-Fluid-Abfalltüte () im Wesentlichen auf derselben Höhe des Membrangasaustauschers (18) oder unterhalb des Membrangasaustauschers (18) platziert ist.

15. Vorrichtung nach einem der Ansprüche 10 bis 14, umfassend eine Einwegkartusche (27) und wobei die Einwegkartusche () den Membrangasaustauscher (18), optional die Blutbehandlungseinheit (2) und mindestens einen Teil des extrakorporalen Blutkreislaufs umfasst.

## Revendications

1. Procédé d'amorçage d'un circuit sanguin extracorporel d'un appareil de traitement extracorporel du sang, l'appareil de traitement extracorporel du sang comprenant :
éventuellement une unité de traitement du sang (2) ;
un circuit sanguin extracorporel, éventuellement couplé à l'unité de traitement du sang (2) ;
une pompe à sang (11) configurée pour être couplée à une section de pompe du circuit sanguin extracorporel ;
un échangeur de gaz à membrane (18) couplé de manière opérationnelle au circuit sanguin extracorporel pour échanger du gaz avec le sang circulant dans le circuit sanguin extracorporel, l'échangeur de gaz à membrane (18) comprenant un côté sang en communication fluide avec le circuit sanguin et un côté gaz ;
le procédé comprenant :
- l'alimentation d'un fluide d'amorçage dans le circuit sanguin extracorporel et dans le côté sang de l'échangeur de gaz à membrane (18) ;
- la génération d'une étape de pressurisation transitoire dans le fluide d'amorçage s'écoulant dans le circuit sanguin et dans le côté sang de l'échangeur de gaz à membrane (18) pour empêcher la libération de bulles d'air au niveau d'une sortie de sang (18d) de l'échangeur de gaz à membrane (18).

2. Procédé selon la revendication 1, comprenant : la répétition de l'étape de pressurisation transitoire pendant l'amorçage, éventuellement à intervalles périodiques.

3. Procédé selon la revendication 1 ou 2, une pression maximale (Pₘₐₓ) au niveau de l'échangeur de gaz à membrane (18) pendant l'étape ou les étapes de pressurisation étant comprise entre 100 mmHg et 1000 mmHg.

4. Procédé selon la revendication 1, 2 ou 3, une durée (Δt) de l'étape de pressurisation ou de chaque étape de pressurisation étant comprise entre 2 secondes et 30 secondes, éventuellement entre 5 s et 10 s.

5. Procédé selon l'une quelconque des revendications 1 à 4, la génération de l'étape de pressurisation transitoire comprenant : la restriction ou l'occlusion transitoire d'une partie du circuit sanguin extracorporel placé en aval de l'échangeur de gaz à membrane (18) par rapport à une direction d'écoulement du fluide d'amorçage.

6. Procédé selon l'une quelconque des revendications 1 à 5, la génération de l'étape de pressurisation transitoire comprenant : le maintien du fonctionnement de la pompe à sang (11) et la fermeture d'une pince (9) placée en aval de l'échangeur de gaz à membrane (18) par rapport à une direction d'écoulement du fluide d'amorçage.

7. Procédé selon l'une quelconque des revendications 1 à 5, la génération de l'étape de pressurisation transitoire étant actionnée par l'intermédiaire d'une ligne de perfusion (15) et d'une pompe à perfusion (17) couplée ou configurée pour être couplée à une section de pompe de la ligne de perfusion (15).

8. Procédé selon l'une quelconque des revendications 1 à 5, la génération de l'étape de pressurisation transitoire étant actionnée par l'intermédiaire d'une chambre de désaération (8) placée sur le circuit sanguin extracorporel et d'une pompe à air (26) raccordée à la chambre de désaération (8).

9. Procédé selon l'une quelconque des revendications 1 à 8, à la fin de l'amorçage et avant la connexion du patient, une pression dans le circuit sanguin et dans le côté sang de l'échangeur de gaz à membrane (18) étant maintenue entre 20 mmHg et 400 mmHg, éventuellement entre 50 mmHg et 100 mmHg.

10. Appareil de traitement extracorporel du sang comprenant :
éventuellement une unité de traitement du sang (2) ;
un circuit sanguin extracorporel, éventuellement couplé à l'unité de traitement du sang (2) ;
une pompe à sang (11) configurée pour être couplée à une section de pompe du circuit sanguin extracorporel ;
un échangeur de gaz à membrane (18) couplé de manière opérationnelle au circuit sanguin extracorporel pour échanger du gaz avec le sang circulant dans le circuit sanguin extracorporel ;
une unité de commande (10) configurée pour commander l'exécution d'une tâche pour amorcer le circuit sanguin extracorporel, ladite tâche comprenant les étapes suivantes :
- alimentation d'un fluide d'amorçage dans le circuit sanguin extracorporel et dans le côté sang de l'échangeur de gaz à membrane (18) ;
- génération d'une étape de pressurisation transitoire dans le fluide d'amorçage s'écoulant dans le circuit sanguin et dans le côté sang de l'échangeur de gaz à membrane (18) pour empêcher la libération de bulles d'air au niveau d'une sortie de sang (18d) de l'échangeur de gaz à membrane (18).

11. Appareil selon la revendication 10, ladite tâche comprenant :
la répétition de l'étape de pressurisation transitoire pendant l'amorçage, éventuellement à des intervalles périodiques.

12. Appareil selon la revendication 10 ou 11, afin de générer l'étape de pressurisation transitoire, ladite tâche comprenant : le maintien du fonctionnement de la pompe à sang (11) et la fermeture, éventuellement la fermeture répétée, d'une pince placée en aval de l'échangeur de gaz à membrane (18) par rapport à une direction d'écoulement du fluide d'amorçage, éventuellement une pince de retour (9).

13. Appareil selon les revendications 10, 11 ou 12, l'échangeur de gaz à membrane (18) étant situé à la suite de l'unité de traitement du sang (2).

14. Appareil selon l'une quelconque des revendications 10 à 13, l'échangeur de gaz à membrane (18) étant situé sensiblement à la même hauteur de l'unité de traitement du sang (2), éventuellement, le sac à déchets de fluide d'amorçage () étant placé sensiblement à la même hauteur de l'échangeur de gaz à membrane (18) ou en dessous de l'échangeur de gaz à membrane (18).

15. Appareil selon l'une quelconque des revendications 10 à 14, comprenant une cartouche jetable (27) et la cartouche jetable () comprenant l'échangeur de gaz à membrane (18), éventuellement l'unité de traitement du sang (2), et au moins une partie du circuit sanguin extracorporel.
